# EUROPÄISCHE PATENTSCHRIFT

(11) **EP 4 240 795 B1**
(45) Veröffentlichungstag und Bekanntmachung des Hinweises auf die Patenterteilung: **30.10.2024**
(21) Anmeldenummer: 20800931.6
(22) Anmeldetag: 04.11.2020
(51) Int. Cl.: C08L 1/28, A61K 49/22, C08K 5/101, C08K 5/11

(54) **KONTAKTMITTEL**
CONTACT MEDIUM
MILIEU DE CONTACT

(43) Veröffentlichungstag der Anmeldung: 13.09.2023
(73) Patentinhaber: Ecco Spray Ltd., Dublin 18 D18NW62 (IE)
(72) Erfinder: Haas, Helmut, 64646 Heppenheim (DE)
(74) Vertreter: WSL Patentanwälte Partnerschaft mbB
(86) Internationale Anmeldenummer: PCT/EP2020/080993
(87) Internationale Veröffentlichungsnummer: WO 2022/096089

(56) Entgegenhaltungen:
- WO-A1-2012/009794
- US-A1- 2014 200 450

## Beschreibung

### Gegenstand und Hintergrund der Erfindung

Die vorliegende Erfindung betrifft ein Kontaktmittel, welches vorzugsweise in der medizinischen Sonographie eingesetzt wird. Das erfindungsgemäße Kontaktmittel ist aber auch in nicht-medizinischen Verfahren zur Untersuchung von technischen Strukturen mittels Ultraschall sowie in anderen medizinischen Verfahren anwendbar, die nicht auf Ultraschall beruhen, aber ein Kontaktmittel erfordern.

Die Sonographie, auch Echografie oder Ultraschalluntersuchung, ist ein bildgebendes Verfahren zur nicht-destruktiven Untersuchung organischer oder technischer Strukturen, welches darauf beruht, dass sich eingekoppelte Schallwellen in unterschiedlichen Medien verschieden schnell ausbreiten und an Grenzflächen unterschiedlicher Wellenimpedanz teilweise reflektiert werden. Die Wellenimpedanz bezeichnet einen Widerstand, der der Ausbreitung der Wellen entgegenwirkt. Mit steigender Differenz der Wellenimpedanz an einer Grenzfläche vergrößert sich auch der reflektierte Anteil der eingekoppelten Wellen. Laufzeit und Stärke der reflektierten Wellen werden detektiert und in Strukturinformationen umgesetzt.

Ein wesentlicher Vorteil der Sonografie in der medizinischen bildgebenden Diagnostik gegenüber beispielsweise Untersuchungen mittels Röntgenstrahlung, z.B. in der Computertomographie (CT), liegt in der Unschädlichkeit der eingesetzten Schallwellen. Auch sind apparativer Aufwand und damit verbundene Kosten sowie räumliche Anforderungen bei der Sonographie in der Regel erheblich geringer als bei der der Computertomographie oder beispielsweise auch der Magnetresonanztomographie, weshalb sonographische Diagnostik, dort wo sie einsetzbar ist und ausreichend aussagekräftige Informationen liefert, auch in kleineren Arztpraxen zur Anwendung kommt.

In der medizinischen Sonographie zur Untersuchung von organischen Strukturen, wie Gewebe, Organen, Blutgefäßen und Knochen, aber auch zur Visualisierung von Blutfluss-Geschwindigkeiten, z.B. mittels Doppler-Verfahren, werden Ultraschallwellen im Frequenzbereich von etwa 1 - 40 MHz eingesetzt. Die Ultraschallwellen werden in einer Sonde erzeugt und in der Regel als kurze, gerichtete Schallwellenimpulse über eine Oberfläche in den Körper eingeleitet. Die reflektierten Wellen werden von einem Detektor, der üblicherweise ebenfalls in der Sonde angeordnet ist, empfangen, in elektronische Signale gewandelt und verarbeitet. Aus der Laufzeit und der Stärke der reflektierten Wellen werden Informationen über die untersuchten Strukturen ermittelt.

Neben Untersuchungen, bei denen eine Sonde z.B. in Körperöffnungen (vaginal, rektal) oder mittels eines Katheters in das Gefäßsystem eingeführt wird, werden die Schallwellenimpulse in den meisten Anwendungen der Sonographie von außen über die Haut in den Körper eingeleitet. Dabei wird der Sondenkopf in der Regel manuell vom untersuchenden Arzt auf der Haut des Patienten aufgesetzt, über diese bewegt und in verschiedenen Winkelstellungen geneigt.

Da der Unterschied der Wellenimpedanz zwischen Luft und Wasser oder organischen Strukturen, einschließlich der Haut, sehr groß ist (Luft: ca 410 kg/m²s; Haut, Fett, Wasser, Muskeln: > 10⁶ kg/m²s), muss die Ultraschallsonde über ein wasserhaltiges Kontaktmittel an die Haut angekoppelt werden, damit der Schall nicht bereits von Lufteinschlüssen zwischen dem Sondenkopf und der Hautoberfläche reflektiert wird, die aufgrund von Unebenheiten und Schuppen der Haut immer vorhanden sind.

Entsprechendes trifft auch auf andere Untersuchungsmethoden zu, bei denen Lufteinschlüsse eine Messung stören können, wie bei Materialprüfungen mittels Ultraschall an nicht-organischen, technischen Strukturen oder auch bei Untersuchungsmethoden, die sehr geringe elektrische Ströme messen, wie EKG- und EEG-Untersuchungen, bei denen Lufteinschlüsse aufgrund des sehr hohen elektrischen Widerstandes eine Messung stark verfälschen oder unmöglich machen würden.

In der medizinischen Sonographie werden als Kontaktmittel zur Ankopplung des Sondenkopfes an die Hautoberfläche überwiegend dickflüssige Gele auf Wasserbasis eingesetzt, die nach verschiedenen Rezepturen hergestellt werden. Solche Gele sind wegen ihrer Inhaltsstoffe teilweise kostenaufwändig in der Herstellung und haben auch andere Nachteile. Beispielsweise wird aufgrund der Dickflüssigkeit regelmäßig mehr Gel als benötigt aufgetragen. Daher ist der Gelverbrauch hoch, einhergehend mit hohen Kosten. Dickflüssige Gele führen bei nicht vollständig entkleideten Patienten häufig zu einer Verschmutzung der Kleidung, und beim Abwischen des Gels wird viel Papier verbraucht, das weitere Kosten verursacht und entsorgt werden muss. Häufig wird das Kontaktmittel vom Patienten als unangenehm kalt empfunden. Die Möglichkeit, das Kontaktmittel vor dem Auftragen auf die Haut des Patienten vorzuwärmen, wäre daher erstrebenswert. Allerdings kann dies bei herkömmlichen dickflüssigen Gelen zu einer Veränderung der physikalischen Eigenschaften führen, welche mit unkontrolliertem Abfluss und Filmabrissen einhergehen. Das Auftragen der dickflüssigen Gele erfolgt meist aus weichwandigen Einmalbehältern aus Kunststoff, die ein Herausdrücken des Gels erlauben. Nach dem Gebrauch werden diese Einmalbehälter in der Regel entsorgt, wobei häufig nicht unerhebliche Mengen an Gel im Behälter verbleiben, die ungenutzt mit verworfen werden. Bei der Applikation des Gels aus der Öffnung des Behälters besteht auch immer die Gefahr, dass die Haut des Patienten damit berührt wird mit der Konsequenz der Keimverschleppung von Patient zu Patient.

Weil der Sondenkopf bei der Sonographie auf der Haut bewegt werden muss, ist neben einer guten Filmbildung bei lückenloser Abdeckung des zu untersuchenden Hautareals auch eine gute Gleitfähigkeit erforderlich. Da dickflüssige Gele diese Anforderungen recht gut erfüllen, werden sie trotz der beschriebenen Nachteile nach wie vor überwiegend eingesetzt. Grundsätzlich wäre aber für eine Ankopplung zwischen Sondenkopf und Haut nur ein dünner Kontaktmittelfilm erforderlich. Die DE 44 34 626 C2 beschreibt ein dünnflüssiges Ultraschallkontaktmittel, das neben Wasser einen Anteil von 0,5-10 Vol.-% Detergentien und 1-25 Vol.-% Ethanol enthält, wobei erst bei einem Anteil von 1 Vol.-% Detergentien und 3-5 Vol.-% Ethanol ein zufriedenstellendes Produkt erhalten wurde, welches einen dauerhaften Flüssigkeitsfilm mit guten Gleiteigenschaften ausbildete und eine Schallwellentransmission aufwies, die derjenigen von handelsüblichen dickflüssigen Kontaktmittelgelen entsprach. Das Kontaktmittel fand jedoch keine Akzeptanz bei Anwendern und Herstellern von Ultraschallgeräten aufgrund des für die ausreichende Filmbildung erforderlichen hohen Ethanolgehalts. Es bestehen Bedenken, dass der Ethanolgehalt bei dauerhafter Anwendung das Material des Sondenkopfs angreift. Zudem führt die Verdunstung des Ethanols in der Anwendung zu einem für den Patienten unangenehmen Kältegefühl sowie zu unerwünschtem

Alkoholgeruch.

Die US2014200450 offenbart ein sprühbares Ultraschallkontaktmittel enthaltend Stärke als Gelbildner.

### Aufgabe der Erfindung

Die Aufgabe der vorliegenden Erfindung bestand daher darin, ein neues Kontaktmittel bereitzustellen, welches die Nachteile bekannter Kontaktmittel insbesondere in der medizinischen Sonographie überwindet oder zumindest verringert.

### Beschreibung der Erfindung

Gelöst wird diese Aufgabe durch ein Kontaktmittel, welches enthält:

| | |
|---|---|
| 0,05 bis 2,5 Gew.-% | Gelbildner |
| 0,05 % bis 3,5 Gew.-% | Detergens |
| 0,1 % bis 4,0 Gew.-% | Emulgiermittel |

optional Zusatzstoffe, vorzugsweise ausgewählt unter Konservierungsmittel, Säuerungsmittel, Hautpflegezusatz und Gemischen davon,
Rest Wasser,
wobei
der Gelbildner aus der Gruppe ausgewählt ist, bestehend aus Hydroxyethylcellulose, Carboxycellulose, Xanthan, Guarkernmehl, Collagen, Gelatine, Pektinen, Chitin, Stärke, Alginaten, einschließlich Natriumalginat, Kaliumalginat und Calciumalginat, und Gemischen davon,
das Detergens ein nicht-ionisches Tensid oder ein Gemisch von nicht-ionischen Tensiden ist,
das Emulgiermittel aus der Gruppe ausgewählt ist, bestehend aus Glycerin-Mono-Fettsäureestern und Glycerin-Di-Fettsäureestern und Gemischen davon, wobei der Fettsäurerest vorzugsweise abgeleitet ist von gesättigter C7-C20-Fettsäure, einfach ungesättigter C16-C20-Fettsäure und/oder zweifach ungesättigter C18-C20-Fettsäure, vorzugsweise von Stearinsäure, Ölsäure, Caprylsäure, Caprinsäure und/oder Linolensäure,
wobei das Kontaktmittel einen pH-Wert im Bereich von 5 bis 7 aufweist.

Das erfindungsgemäße Kontaktmittel ist im Vergleich zu handelsüblichen Kontaktgelen dünnflüssig, es besitzt somit eine niedrigere Viskosität bzw. höhere Fließfähigkeit. Die Viskosität bzw. Fließfähigkeit des erfindungsgemäßen Kontaktmittels lässt sich durch die Auswahl der eingesetzten Mengen der Bestandteile, insbesondere des Gelbildners, einstellen. Die Bestimmung der Fließfähigkeit des erfindungsgemäßen Kontaktmittels erfolgte hierin gemäß ISO 2431 (DIN EN ISO 2431:2020-02) durch Messung der Auslaufzeit eines Messvolumens von 100 ml Kontaktmittelzusammensetzung unter Verwendung eines ISO-Auslaufbechers Nr. 4 mit einem Düsendurchmesser von 4 mm bei Raumtemperatur (25 °C) sowie gegebenenfalls bei Körpertemperatur (37 °C). In einer bevorzugten Ausführungsform besitzt das erfindungsgemäße Kontaktmittel eine auf diese Weise bestimmte Auslaufzeit bei 25°C im Bereich von 5 bis 200 Sekunden, vorzugsweise im Bereich von 10 bis 150 Sekunden, besonders bevorzugt im Bereich von 10 bis 100 Sekunden, ganz besonders bevorzugt im Bereich von 10 bis 50 Sekunden.

Ist die Viskosität des erfindungsgemäßen Kontaktmittels zu gering, wird keine ausreichende Filmbildung erzielt und es besteht die Gefahr von Filmabrissen. Ist die Viskosität des erfindungsgemäßen Kontaktmittels zu hoch, treten die beschriebenen Nachteile dickflüssiger Kontaktmittelgele auf.

Bei Anwendung in der medizinischen Sonographie bildet das erfindungsgemäße Kontaktmittel trotz seiner Dünnflüssigkeit bzw. niedrigen Viskosität auf der Haut einen dünnen Kontaktmittelfilm mit einer guten und lückenlosen Abdeckung des behandelten Hautareals ohne signifikante Filmabrisse. Gleichzeitig stellt es eine gute Gleitfähigkeit des Sondenkopfs auf der Haut sicher. Die Schallwellentransmission des erfindungsgemäßen Kontaktmittels ist mit derjenigen von handelsüblichen dickflüssigen Kontaktmittelgelen vergleichbar, teilweise je nach Zusammensetzung sogar besser.

Darüber hinaus lässt sich das erfindungsgemäße Kontaktmittel im Vergleich zu handelsüblichen Kontaktgelen leicht und aufgrund der vergleichsweise geringen erforderlichen Einsatzmenge mit weniger Papieraufwand wieder von der Haut entfernen.

Ein weiterer besonderer Vorteil des erfindungsgemäßen Kontaktmittels besteht darin, dass es mit einem für dünnflüssige Medien geeigneten Applikator aufgebracht werden kann, beispielsweise mit einer handelsüblichen Sprühflasche. Die Sprühflasche kann nach Verbrauch des Kontaktmittels wieder befüllt und erneut verwendet werden, wodurch Verpackungsabfall vermieden werden kann. Die Applikation des Kontaktmittels mit einer Sprühflasche vermeidet zudem die Berührung der Haut des Patienten und vermindert so die Gefahr der Verschleppung von Keimen von einem Patienten zum nächsten. Das Kontaktmittel kann beispielsweise aus großen Gebinden nachgefüllt werden. Es bleibt somit auch kein nicht mehr verwendbares Kontaktmittel in einem Einwegapplikator zurück, das mit diesem entsorgt werden müsste, wie es bei dickflüssigen Kontaktmittelgelen regelmäßig der Fall ist, die in Einwegapplikatoren vertrieben und verwendet werden.

Darüber hinaus kann das erfindungsgemäße Kontaktmittel aufgrund der Verwendung preiswerter Inhaltstoffe kostengünstiger als handelsübliche Kontaktmittelgele hergestellt werden.

Das erfindungsgemäße Kontaktmittel kann den Gelbildner in einer Menge von 0,05 bis 2,5 Gew.-% enthalten. Mit höheren Anteilen des Gelbildners im Bereich von über 0,5 bis 2,5 Gew.-% kann das Kontaktmittel je nach verwendetem Gelbildner bereits eine Viskosität besitzen, die sich der eines handelsüblichen Kontaktgels annähert, wobei das Kontaktmittel dann aber immer noch dünn aufgetragen werden kann und die oben beschriebenen Vorteile hat.

In einer besonders bevorzugten Ausführungsform enthält das erfindungsgemäße Kontaktmittel jedoch den Gelbildner in einer Menge von 0,05 bis 0,5 Gew.-%. Ein solches erfindungsgemäßes Kontaktmittel ist besonders dünnflüssig, besitzt aber dennoch alle hierin beschriebenen Vorteile und ist insbesondere sehr gut applizierbar, beispielsweise mittels einer Sprühflasche. Zudem ist es kostengünstiger herstellbar, da weniger Gelbildner benötigt wird.

Neben Gelbildner, Detergens und Emulgiermittel kann das erfindungsgemäße Kontaktmittel optional weitere Zusatzstoffe enthalten, wie Konservierungsmittel, Puffer- oder Säuerungsmittel, Hautpflegezusatz etc. Solche Zusatzstoffe können mit Vorteil eingesetzt werden, jedoch in so geringen Mengen, dass sie die vorteilhaften Eigenschaften des Kontaktmittels nicht oder zumindest nicht signifikant beeinträchtigen, wie Fließ- und Filmbildungseigenschaften, Gleitfähigkeit sowie die Schallwellentransmission.

In einer Ausführungsform enthält das erfindungsgemäße Kontaktmittel ein Konservierungsmittel vorzugsweise in einer Menge von 0,05 bis 1,0 Gew.-%, wobei das Konservierungsmittel besonders bevorzugt aus der Gruppe ausgewählt ist, bestehend aus Natrium-Benzoat, Kalium-Benzoat, Natrium-Sorbat, Kalium-Sorbat und Gemischen davon. Ein zu geringer Anteil an Konservierungsmittel hat keine oder eine zu geringe Konservierungswirkung. Ein zu hoher Anteil bringt keine wesentliche zusätzliche Konservierungswirkung und/oder kann die physikalischen und/oder chemischen Eigenschaften des Kontaktmittels nachteilig beeinflussen. In einer vorteilhaften Ausführungsform der Erfindung enthält das Kontaktmittel ein Gemisch aus etwa 0,3 bis 0,5 Gew.-% Natrium-Benzoat und etwa 0,1 bis 0,3 Gew.-% Kalium-Sorbat. Der Zusatz des Konservierungsmittels verbessert die Haltbarkeit und Lagerungsfähigkeit des erfindungsgemäßen Kontaktmittels, ohne die weiteren vorteilhaften Eigenschaften signifikant zu beeinträchtigen.

Das erfindungsgemäße Kontaktmittel weist einen pH-Wert im Bereich von 5 bis 7 auf. Es ist damit bereits gut hautverträglich. Zudem hat sich gezeigt, dass ein pH-Wert im Bereich von 5 bis 7 die durch die erfindungsgemäße Kombination von Gelbildner, Detergens und Emulgiermittel erzielten vorteilhaften physikalischen und chemischen Eigenschaften des Kontaktmittels stabilisiert. In einer weiteren vorteilhaften Ausführungsform der Erfindung weist das Kontaktmittel einen pH-Wert im Bereich von 5 bis 6,5, vorzugsweise im Bereich von 5 bis 6, besonders bevorzugt im Bereich von 5 bis 5,6 auf. Ein niedrigerer pH-Wert im Bereich von 5 bis 6 oder sogar ≤ 5,6 ist in vielen Fällen vorteilhaft, da dadurch unter anderem bei Gegenwart von Konservierungsmittel eine bessere Konservierungswirkung als bei höheren pH-Werten erzielt wird.

Je nach Auswahl und Mengen an eingesetztem Gelbildner, Detergens und Emulgiermittel sowie Zusatzstoffen kann es erforderlich sein, den pH-Wert abzusenken und/oder zu puffern. In einer Ausführungsform enthält das erfindungsgemäße Kontaktmittel daher zum Einstellen und/oder Puffern des pH-Wertes ein anorganisches oder organisches Puffersystem oder Säuerungsmittel. Ein erfindungsgemäß geeignetes Säuerungsmittel ist Zitronensäure. Erfindungsgemäß geeignete Puffersysteme sind Natriumzitrat-Zitronensäure-Puffer oder Phosphat-Zitrat-Puffer, beispielsweise Dinatriumphosphat-Zitronensäure-Puffer. Die erforderlichen Mengen an Puffer- oder Säuerungsmittel sind gering und vom Fachmann routinemäßig zu ermitteln. In Versuchen hat sich beispielsweise eine Menge von 2 Gew.-% Zitronensäure als geeignet erwiesen, den pH-Wert einer erfindungsgemäßen Zusammensetzung auf 5,6 zu senken.

Weiterhin kann das erfindungsgemäße Kontaktmittel mit Vorteil einen Hautpflegezusatz enthalten. Geeignete Hautpflegezusätze sind dem Fachmann aus der Kosmetik sowie aus medizinischen und nicht-medizinischen Hautpflegemitteln bekannt. Ein erfindungsgemäß vorteilhafter Hautpflegezusatz ist beispielsweise Aloe Vera alleine oder im Gemische mit anderen Hautpflegezusätzen. Die zugegebene Menge an Hautpflegezusatz ist routinemäßig so zu wählen, dass eine gewünschte Hautpflegewirkung erzielt wird, ohne die vorteilhaften Eigenschaften des erfindungsgemäßen Kontaktmittels signifikant zu beeinträchtigen.

Die wesentlichen Bestandteile des erfindungsgemäßen Kontaktmittels, welche in ihrer Kombination die für die hierin beschriebenen Anwendungen vorteilhaften physikalischen und chemischen Eigenschaften des Kontaktmittels erzielen, sind Gelbildner, Detergens und Emulgiermittel.

Ein erfindungsgemäß besonders geeigneter und bevorzugter Gelbildner ist Hydroxyethylcellulose, vorzugsweise 2-Hydroxyethylcellulose (CAS# 9004-62-0) mit einem durchschnittlichen Substitutionsgrad (DS) von 0,85 - 1,35 und/oder einem molaren Substitutionsgrad (MS) von 1,5 - 3,0. Der Gelbildner kann alleine oder im Gemisch mit weiteren der vorgenannten Gelbildner eingesetzt werden. Als Gelbildner erfindungsgemäß besonders geeignete Hydroxyethylcellulose ist handelsüblich unter den Bezeichnungen Natrosol^{®} (Ashland Inc.), Cellosize^{®} (Dow Chemical) und Tylose^{®} (Shin-Etsu) erhältlich. In den hierin nachstehend beschriebenen Beispielen wurde das Produkt Natrosol^{®} 250 HX eingesetzt.

In einer bevorzugten Ausführungsform des erfindungsgemäßen Kontaktmittels sind mehr als 80 Gew.-%, vorzugsweise mehr als 90 Gew.-% des Gelbildners Hydroxyethylcellulose. In einer weiteren bevorzugten Ausführungsform des erfindungsgemäßen Kontaktmittels ist der gesamte Gelbildner Hydroxyethylcellulose.

In einer vorteilhaften Ausführungsform enthält das erfindungsgemäße Kontaktmittel 0,1 bis 0,3 Gew.-% Gelbildner. Es konnte überraschend gezeigt werden, dass eine derart geringe Menge an Gelbildner ausreichen kann, um in Kombination mit erfindungsgemäßem Detergens und Emulgiermittel ein Kontaktmittel zu erhalten mit den hierin beschriebenen Eigenschaften und Vorteilen, insbesondere ausgezeichnete Filmbildungseigenschaften, Gleitfähigkeit und Schallwellentransmission.

Das Detergens ist ein nicht-ionisches Tensid oder ein Gemisch von nicht-ionischen Tensiden. In Kenntnis der Erfindung ist der Fachmann in der Lage, geeignete nicht-ionische Tenside auszuwählen. Bevorzugt ist das Detergens aus der Gruppe ausgewählt, bestehend aus polyethoxyliertem Kastoröl, Poloxameren und Gemischen davon. In einer bevorzugten Ausführungsform des erfindungsgemäßen Kontaktmittels sind mehr als 80 Gew.-%, vorzugsweise mehr als 90 Gew.-% des Detergens polyethoxyliertes Kastoröl. In einer weiteren bevorzugten Ausführungsform des erfindungsgemäßen Kontaktmittels ist das gesamte Detergens polyethoxyliertes Kastoröl, welches handelsüblich unter den Bezeichnungen Cremophor^{®} oder Kolliphor^{®} erhältlich ist. In den hierin nachstehend beschriebenen Beispielen wurde das Produkt Kolliphor ^{®} RH40 (Synonym Cremophor^{®} RH40; Macrogol-Glycerol-Hydroxystearat, PEG-40 Kastoröl; CAS Nummer: 61788-85-0; Merck KGaA, Darmstadt, Deutschland) eingesetzt.

In einer vorteilhaften Ausführungsform enthält das erfindungsgemäße Kontaktmittel 0,075 % bis 0,5 Gew.-% Detergens. Es konnte überraschend gezeigt werden, dass eine derart geringe Menge an Detergens ausreichen kann, um in der erfindungsgemäßen Zusammensetzung ein Kontaktmittel zu erhalten mit den hierin beschriebenen Eigenschaften und Vorteilen, insbesondere ausgezeichnete Filmbildungseigenschaften, Gleitfähigkeit und Schallwellentransmission.

Das Emulgiermittel ist aus der Gruppe ausgewählt ist, bestehend aus Glycerin-Mono-Fettsäureestern und Glycerin-Di-Fettsäureestern und Gemischen davon, wobei der Fettsäurerest vorzugsweise abgeleitet ist von gesättigter C7-C20-Fettsäure, einfach ungesättigter C16-C20-Fettsäure und/oder zweifach ungesättigter C18-C20-Fettsäure, vorzugsweise von Stearinsäure, Ölsäure, Caprylsäure, Caprinsäure und/oder Linolensäure.

In einer bevorzugten Ausführungsform des erfindungsgemäßen Kontaktmittels sind mehr als 80 Gew.-%, vorzugsweise mehr als 90 Gew.-% des Emulgiermittels Glycerin-Mono-Fettsäureester. In einer weiteren bevorzugten Ausführungsform des erfindungsgemäßen Kontaktmittels ist das gesamte Emulgiermittel Glycerin-Mono-Fettsäureester. Erfindungsgemäß geeignete handelsübliche Emulgiermittel sind unter der Bezeichnung Imwitor^{®} erhältlich. In den hierin nachstehend beschriebenen Beispielen wurde das Produkt Imwitor^{®} 742 eingesetzt, ein Glycerin-Mono-Caprylocaprat (Macrogol-6-glycerol-caprylocaprat), erhältlich von IOI Oleo GmbH, Hamburg, Deutschland.

In einer vorteilhaften Ausführungsform enthält das erfindungsgemäße Kontaktmittel 0,2 Gew.-% bis 0,8 Gew.-% Emulgiermittel. Es konnte überraschend gezeigt werden, dass eine derart geringe Menge an Emulgiermittel ausreichen kann, um in der erfindungsgemäßen Zusammensetzung ein Kontaktmittel zu erhalten mit den hierin beschriebenen Eigenschaften und Vorteilen, insbesondere ausgezeichnete Filmbildungseigenschaften, Gleitfähigkeit und Schallwellentransmission.

In einer weiteren vorteilhaften Ausführungsform enthält das erfindungsgemäße Kontaktmittel 0,1 bis 0,3 Gew.-% Gelbildner, 0,075 bis 0,5 Gew.-% Detergens und 0,2 bis 0,8 Gew.-% Emulgiermittel.

Das erfindungsgemäße Kontaktmittel entwickelt seine vorteilhaften Eigenschaften trotz des geringem Stoffeinsatzes und damit preisgünstiger Herstellbarkeit und seiner Dünnflüssigkeit bzw. niedrigen Viskosität gegenüber handelsüblichen Kontaktmittelgelen vollständig auf Wasserbasis. Der Zusatz eines einwertigen Alkanols, insbesondere von Ethanol, als zusätzlicher Bestandteil ist nicht erforderlich. Das erfindungsgemäße Kontaktmittel hat daher auch Vorteile gegenüber bekanntem dünnflüssigem Kontaktmittel, welches einen Anteil von 3 bis 5 Vol.-% Ethanol erfordert, um ein hinsichtlich Filmbildung und Schallwellentransmission zufriedenstellendes Produkt zu erhalten, aber wegen des Ethanolanteils keine Akzeptanz bei Anwendern und Herstellern von Ultraschallgeräten findet. Ein geringer Anteil Ethanol ist bei dem erfindungsgemäßen Kontaktmittel nicht störend, bringt aber keine weiteren Vorteile, sondern eher die bekannten Nachteile. Das erfindungsgemäße Kontaktmittel enthält daher zweckmäßigerweise höchstens 1 Gew.-%, vorzugsweise höchstens 0,5 Gew.-% eines einwertigen Alkanols und besonders bevorzug kein einwertiges Alkanol.

Die vorteilhaften Eigenschaften des erfindungsgemäßen Kontaktmittels wurden nur mit der Kombination der erfindungsgemäßen Bestandteile Gelbildner, Detergens und Emulgiermittel in den erfindungsgemäßen Konzentrationen erzielt. Die chemischen und physikalischen Eigenschaften jedes einzelnen dieser Bestandteile waren grundsätzlich bekannt. Nicht zu erwarten und daher überraschend war jedoch ihr Zusammenwirken in der erfindungsgemäßen Kombination und in den erfindungsgemäß niedrigen Konzentrationen zu einem Kontaktmittel mit hervorragenden Eigenschaften insbesondere in der Sonographie. Die Wirkung der Bestandteile in dem erfindungsgemäßen Kontaktmittel geht über einen rein additiven Effekt der Eigenschaften der einzelnen Bestandteile deutlich hinaus. Dies zeigen die Ergebnisse der nachfolgenden Beispiele. Tatsächlich war aufgrund der bekannten Eigenschaften der einzelnen Bestandteile bei einer Kombination der Bestandteile eher mit Nachteilen zu rechnen. Es wird daher angenommen, dass die vorteilhaften Eigenschaften des erfindungsgemäßen Kontaktmittels auf einem synergistischen Effekt der Bestandteile beruhen, der so nicht zu erwarten war. Versuche, bei denen einzelne Bestandteile ausgelassen oder in Konzentrationen außerhalb der erfindungsgemäßen Bereiche eingesetzt wurden, bestätigen den Effekt.

Beispielsweise werden in herkömmlichen Kontaktmittelgelen die Ausbildung eines zusammenhängenden Kontaktmittelfilms und gute Gleitfähigkeit des Sonographie-Sondenkopfes auf der Haut im Wesentlichen den hohen Konzentrationen an Gelbildner zugeschrieben. Bei erheblich geringeren Konzentrationen an Gelbildner, um eine dünnflüssigere bzw. niedriger viskose Konsistenz als bei herkömmlichen Kontaktmittelgelen zu erzielen, musste zunächst mit schlechteren Filmbildungs- und Gleiteigenschaften gerechnet werden. Tatsächlich wurde bei den erfindungsgemäß niedrigen Konzentrationen an Gelbildner ohne die beiden weiteren Bestandteile Detergens und Emulgiermittel sogar eine Ausbildung von kugeligen Geltropfen sowie Schaumbildung beobachtet, was der Eignung als Kontaktmittel für die Sonographie entgegen stand. Die Hinzugabe von erfindungsgemäßem Detergens reduzierte zwar die Bildung der kugeligen Geltropfen, was möglicherweise auf eine Veränderung von Oberflächenspannungen zurückzuführen war, allerdings waren die Filmbildungseigenschaften weiterhin schlecht. Zudem bildeten sich erhebliche Mengen an Luftbläschen (Schaumbildung), die diese Kombination aus Gelbildner und Detergens für eine Verwendung als Kontaktmitttel in der Sonographie unbrauchbar erscheinen ließ, da das Kontaktmittel ja gerade die störenden Einflüsse von Lufteinschlüssen bei der Einkopplung der Schallwellen in die Haut ausräumen soll. Die Zugabe von Emulgiermittel alleine zum Gelbildner verbesserte geringfügig die Filmbildungseigenschaften, aber es wurden nach wie vor Tropfen- und Schaumbildung beobachtet. Erst in der erfindungsgemäßen Kombination aus Gelbildner, Detergens und Emulgiermittel wurden trotz der geringen Konzentrationen besonders gute Filmbildungs- und Gleiteigenschaften und eine deutliche Minderung bzw. ein Verschwinden der störenden Gasbläschen und eine gute Schallwellentransmission festgestellt.

Bei geringem Stoffeinsatz und damit preisgünstiger Herstellbarkeit wurde durch die erfindungsgemäße Kombination ein Kontaktmittel erhalten, das vergleichbare oder sogar bessere Schallwellentransmissionseigenschaften als handelsübliche Kontaktgele aufweist und gleichzeitig aufgrund seiner Dünnflüssigkeit Vorteile in der Handhabung und im Verbrauch besitzt, wie Sprühbarkeit, und eine lückenlose Abdeckung des behandelten Hautareals ohne signifikante Filmabrisse sowie gute Gleitfähigkeit. Die vorteilhaften Eigenschaften des erfindungsgemäßen Kontaktmittels bleiben auch erhalten, wenn das Kontaktmittel erwärmt wird, zumindest bis zu einer Temperatur, bei der es der Patient als angenehm empfindet, beispielsweise 37°C (Körpertemperatur). Hinzu kommt, dass bei Verwendung eines aus der Distanz gesprühten Kontaktmittels die Gefahr einer Keimverschleppung äußerst gering ist.

### Beispiele

In Versuchsreihen wurden unterschiedliche Zusammensetzungen aus Gelbildner, Detergens und/oder Emulgiermittel einzeln und in verschiedenen Kombinationen sowie in unterschiedlichen Konzentrationen hergestellt und hinsichtlich ihrer Eigenschaften überprüft, wie Filmbildung, Benetzung, Dauerhaftigkeit des Kontaktmittelfilms, Ausbildung von Gasblasen, Sprühbarkeit aus Pumpsprayflaschen, Gleiteigenschaften (u.a. Sondenkopfgleitfähigkeit auf der Haut), Eignung als Sonographie-Kontaktmittel (Schallwellentransmission). Diese Eigenschaften der Zusammensetzungen wurden sowohl anhand eines "In-vitro"-Modells als auch eines "In-vivo"-Modells am Patienten überprüft.

### In-vitro-Modell

Im In-vitro-Modell wurde die Haut eines Patienten durch eine handelsübliche Büro-Folienhülle mit einer leicht rauhen Oberfläche und die Kontaktfläche eines Sondenkopfs durch eine Folienhülle mit besonders glatter Oberfläche simuliert. Ausbildung, Gleichmäßigkeit und Dauerhaftigkeit des Kontaktmittelfilms wurden auf der Büro-Folienhülle mit leicht rauher Oberfläche getestet. Die Gleichmäßigkeit des Kontaktmittelfilms wurde visuell beurteilt, die Dauerhaftigkeit durch Beobachtung über die Zeit. Die Gleiteigenschaften des Kontaktmittels wurden zudem durch Verreiben der Zusammensetzungen mit PE-Einmalhandschuhen getestet und vergleichend bewertet. Die Bildung bzw. das Fehlen von Gasblasen wurde visuell beurteilt.

Die Sprühfähigkeit wurde durch Beobachtung des beim Sprühen der Zusammensetzung aus einer handelsüblichen Sprühflasche aus der Düse austretenden Sprühkegels bewertet. Bei einem Sprühkegelwinkel von etwa 30° wurde eine gute Sprühbarheit angenommen und bei einem Sprühkegelwinkel von 50° eine sehr gute Sprühbarkeit.

### In-vivo-Modell

Im "In-vivo"-Modell am Patienten wurden die Eigenschaften der getesteten Zusammensetzungen als Kontaktmittel wie bei einer üblichen Sonographie geprüft. Die Schalltransmissionseigenschaften der Kontaktmittel wurden durch Vergleich erhaltener Sonographie-Bilder gegenüber solchen, die mit einem in der medizinischen Sonographie verwendeten handelsüblichen Kontaktmittelgel (Sonogel, Fa. Sonogel Vertriebs GmbH) erhalten wurden, visuell bewertet. Dabei wurden bei demselben Patienten jeweils markante Organstrukturen desselben Organs mit verschiedenen Zusammensetzungen des Kontaktmittels dargestellt.

Die blasenfreie Ausbildung eines gleichmäßigen Flüssigkeitsfilms und die Gleitfähigkeit des Sondenkopfs wurden auch im In-vivo-Modell am Patienten beurteilt, ebenso die Mitnahme eines Flüssigkeitsfilms beim Überschreiten der Grenzen des benetzten Bereichs.

### Herstellung von Zusammensetzungen

In den nachfolgend beschriebenen Versuchen wurden folgende Bestandteile zur Herstellung von Zusammensetzungen verwendet:

| | | |
|---|---|---|
| a) | Gelbildner: | Hydroxyethylcellulose (Natrosol^{®} 250HX) |
| b) | Detergens: | polyethoxyliertes Kastoröl (Kolliphor^{®} RH40, CAS# 61788-85-0) |
| c) | Emulgiermittel: | Glycerin-Mono-Caprylocaprat (Imwitor^{®} 742) |

Der Gelbildner Natrosol liegt als Pulver vor, während das Detergens Kolliphor und das Emulgiermittel Imwitor als wachsartige Massen vorliegen. Imwitor wird vor Produktionsbeginn für etwa 12 Stunden im Wärmeschrank bei 60°C homogenisiert. Kolliphor und Wasser (H₂O dest.) werden ebenfalls auf 60°C vorgewärmt. Unter Rühren wird das auf 60°C vorgewärmte Imwitor zunächst in das Wasser eingerührt und dann Kolliphor unter Rühren zugegeben. Anschließend wird das pulverförmige Natrosol unter starkem Rühren zur Vermeidung von Klumpenbildung in geringen Dosen nach und nach eingebracht. Die Zusammensetzung wird gerührt, bis sie homogen ist, und anschließend abkühlen und ruhen gelassen, bis sich bei der Herstellung gebildeter Schaum zersetzt hat. Die Zusammensetzung kann dann abgefüllt und ggf. in einem Autoklaven sterilisiert werden.

Zusatzstoffe, wie Konservierungsmitel etc., können nach der Zugabe des Kolliphors und/oder nach dem Einrühren des Natrosols eingebracht und unter Rühren gelöst werden. Der pH-Wert kann nach der Zugabe des Kolliphors und/oder nach dem Einrühren des Natrosols bei laufender pH-Wert-Messung durch Zugabe von Säure oder Puffer, z. B. Zitronensäure, eingestellt werden. Zum Erreichen eines pH-Wertes von etwa 5,5-5,6 wurden bei einer üblichen Zusammensetzung etwa 1,5-2,0 Gew.-% Zitronensäure eingesetzt.

### Beispiel 1

In Testreihen wurden zunächst einzelne der Komponenten (Gelbildner, Detergens und Emulgiermittel), Kombinationen aus jeweils zwei der Komponenten sowie Kombinationen aus allen drei der erfindungsgemäßen Komponenten in unterschiedlichen Zusammensetzungen hinsichtlich unterschiedlicher Eigenschaften (A bis I) untersucht und bewertet. Die untersuchten und bewerteten Eigenschaften (A bis I) sowie die Bewertungskriterien sind in der nachfolgenden Tabelle 1 zusammengefasst. Die Ergebnisse sind in den nachfolgenden Tabellen 2 und 3 wiedergegeben.

**Tabelle 1: Tests A bis I sowie Bewertungskriterien**

| **Tests** | | **Bewertungskriterien** | |
|---|---|---|---|
| **A** | "Schaumbildung" | | |
| | Die Mischung wurde in einer Flasche kräftig geschüttelt, die Flasche 2 min stehen gelassen und die Schaumbildung in der Flasche visuell beurteilt. | - | stark |
| | | + | mittel |
| | | ++ | schwach |
| | | +++ | kein Schaum |
| **B** | "Filmbildung" | | |
| | Die Mischung wurde auf eine raue Folie aufgesprüht und die Gleichmäßigkeit des Films bzgl. Randschärfe und Homogenität visuell beurteilt. | - | kein Film |
| | | + | unregelmäßig |
| | | ++ | teils unregelmäßig |
| | | +++ | gleichmäßig |
| **C** | "Tropfenbildung" | | |
| | Nach Aufsprühen der Mischung auf eine raue Folie wurde die Bildung und Ausprägung von Tropfen visuell beurteilt. | - | große Tropfen |
| | | + | kleine Tropfen |
| | | ++ | keine Tropfen |
| **D** | "Verweildauer Film / Bläschen" | in Stunden (h) | |
| | Nach Aufsprühen der Mischung auf eine raue Folie wurde die Verweildauer des aufgespühten Films oder der gebildeten Bläschen bestimmt. | | |
| **E** | Ösentest" | | |
| | Eine (Draht-) Öse (Durchmesser 2 cm) wurde in die Mischung eingetaucht, herausgehoben und die Dauer (Haltbarkeit) des Films in der Öse bestimmt. Die Haltbarkeit des Films in der Öse gibt Aufschluss über die Oberflächenspannung der Mischung. | - | < 1 sec |
| | | + | 2 -5sec |
| | | ++ | > 5 sec |
| | | +++ | > 10 sec |
| **F** | "Gleitfähigkeit" | | |
| | Die Mischung wurde auf eine raue Folie aufgesprüht und die Gleitfähigkeit über das Verhalten beim Verreiben mit einem PE-Folienhandschuh beurteilt. | + | schwach |
| | | ++ | mittel |
| | | +++ | gut |
| **G** | "Besonderes" | | |
| | Besonderheiten bei den Tests | | Beschreibung |
| Kontrolle der Mischungen in der Flasche nach 10 Tagen | | | |
| **H** | "Gelschlieren" | | |
| | Die Mischung wurde für 10 Tage in einer Flasche stehen gelassen und anschließend die Bildung abgesetzter Gelschlieren am Flaschenboden beurteilt | - | mittel |
| | | + | schwach |
| | | ++ | nicht vorhanden |
| **I** | Schaumbildung nach 10 Tg." | | |
| | Die Mischung wurde für 10 Tage in einer Flasche stehen gelassen und die Schaumbildung wie im Test A beurteilt. | - | stark |
| | | + | schwach |
| | | ++ | nicht vorhanden |

**Tabelle 2: Einzelsubstanzen und Zweierkombinationen mit Natrosol**

| | Emulgiermittel | Detergens | Gelbildner | **A** | **B** | **C** | **D** | **E** | **F** | **G** |
|---|---|---|---|---|---|---|---|---|---|---|
| Nr. (#) | Imwitor Gew.-% | Kolliphor Gew.-% | Natrosol Gew.-% | Schaumbildung | Filmbildung | Tropfenbildung | Verweildauer | ÖsenTest | Gleitfähigkeit | Besonderes |
| 1 | 2,0 | | | +++ | + | ++ | 1 Std. | - | +++ | |
| 2 | | 2,0 | | ++ | + | + | 2 - 3 Std. | + | ++ | |
| 3 | | | 0,2 | +++ | - | - | 3 Std. + | ++ | ++ | Perlen |
| 4 | | | 0,4 | +++ | - | - | 3 Std. + | +++ | ++ | Perlen |
| 5 | | | 0,6 | +++ | - | - | 3 Std. + | +++ | +++ | Perlen |
| 6 | 1,0 | | 0,2 | +++ | + | ++ | 1 Std.+ | - | +++ | Kegel 20° |
| 7 | | 1,0 | 0,2 | ++ | - | + | 2 Std.+ | ++ | +++ | Strahl |

**Tabelle 3: Kombinationen von Emulgiermittel, Detergens und Gelbildner**

| | Emulgiermittel | Detergens | Gelbildner | **A** | **B** | **C** | **D** | **E** | **F** | **G** | **H** | **I** |
|---|---|---|---|---|---|---|---|---|---|---|---|---|
| Nr. (#) | Imwitor Gew.-% | Kolliphor Gew.-% | Natrosol Gew.-% | Schaumbildung | Filmbildung | Tropfenbildung | Verweildauer | ÖsenTest | Gleitfähigkeit | Besonderes | Gelschlie ren (10 Tg.) | Schaumbildung (10 Tg.) |
| 8 | 1,0 | 1,0 | 0,4 | ++ | ++ | + | 2 - 3 Std. | ++ | ++ | | - | ++ |
| 9 | 1,0 | 1,5 | 0,4 | + | + | ++ | 2 - 3 Std. | + | +++ | | - | ++ |
| 10 | 1,0 | 2,0 | 0,4 | + | + | ++ | 2 - 3 Std. | - | ++ | | - | ++ |
| 11 | 0,66 | 1,33 | 0,27 | - | + | + | 2 - 3 Std. | ++ | + | sehr viel Schaum | ++ | + |
| 12 | 1,0 | 1,0 | 0,2. | - | + | ++ | 2 - 3 Std. | - | +++ | Bodenschlieren | - | ++ |
| 13 | 1,0 | 1,5 | 0,2 | - | + | ++ | 2 - 3 Std. | ++ | +++ | | + | + |
| 14 | 1,0 | 2,0 | 0,2 | - | ++ | ++ | 2 - 3 Std. | +++ | +++ | | ++ | + |
| 15 | 2,0 | 1,0 | 0,4 | ++ | + | + | 2 - 3 Std. | - | ++ | | + | ++ |
| 16 | 2,0 | 1,5 | 0,4 | + | + | ++ | 2 - 3 Std. | ++ | ++ | | ++ | ++ |
| 17 | 2,0 | 2,0 | 0,4 | + | ++ | + | 2 - 3 Std. | +++ | ++ | | - | + |
| 18 | 1,3 | 3 | 0,3 | + | + | ++ | 2 - 3 Std. | + | +++ | | + | ++ |
| 19 | 0,89 | 3,33 | 0,2 | + | +++ | - | 2 - 3 Std. | + | +++ | viele Bläschen | ++ | - |
| 20 | 2,0 | 1,0 | 0,2 | ++ | ++ | ++ | 2 - 3 Std. | - | +++ | | ++ | ++ |
| 21 | 2,0 | 1,5 | 0,2 | + | + | (+) | 2 - 3 Std. | ++ | ++ | Bodenschlieren | - | ++ |
| 22 | 2,0 | 2,0 | 0,2 | + | + | ++ | 2 - 3 Std. | (+) | +++ | Sprühstrahl, kein Kegel, Bläschen | - | ++ |

### Beispiel 2

Unter den Zusammensetzungen aus Beispiel 1, Tabelle 3 wurden vielversprechende Mischungen ausgewählt, weiter verdünnt und "in vivo" auf der Haut eines Patienten überprüft. Die Schalltransmissionseigenschaften waren bei allen Mischungen gleich gut.

Die ausgewählten Zusammensetzungen wurden hinsichtlich unterschiedlicher Eigenschaften (J bis N) untersucht und bewertet. Als Vergleichszusammensetzung wurde ein handelsübliches Kontaktmittelgel (Sonogel, Fa. Sonogel Vertriebs GmbH) eingesetzt. Die untersuchten und bewerteten Eigenschaften (J bis N) sowie die Bewertungskriterien sind in der nachfolgenden Tabelle 4 zusammengefasst, und die Ergebnisse sind in der nachfolgenden Tabelle 5 wiedergegeben.

**Tabelle 4: Tests J bis I sowie Bewertungskriterien**

| **Tests auf der Haut** | | **Bewertungskriterien** | |
|---|---|---|---|
| **J** | Sprüheigenschaft "Sprühkegel" | | |
| | Die Mischung wurde mit einem einfachen Sprühkopf versprüht. Anhand einer Schablone wurde der Winkel des Sprühkegels geschätzt oder das Auftreten lediglich eines Sprühstrahls ohne Kegelbildung registriert. | "##" | geschätzter Winkelgrad Sprühkegel |
| | | 0 | Sprühstrahl ohne Kegel |
| **K** | "Filmbildung - Haut" | | |
| | Nach Aufsprühen der Mischung auf die Haut wurde die Ausbildung eines homogenen Films beurteilt. | - | nein |
| | | + | eingeschränkt |
| | | +++ | ja |
| **L** | "Tropfenbildung - Haut" | | |
| | Nach Aufsprühen der Mischung auf die Haut wurde die Bildung von Tropfen visuell beurteilt. | - | ja |
| | | +++ | nein |
| **M** | "Gleitfähigkeit - Haut" | | |
| | Nach Aufsprühen der Mischung auf die Haut wurde die Gleitfähigkeit des Sondenkopfs innerhalb des aufgebrachten Films beurteilt. | + | eingeschränkt |
| | | ++ | mäßig |
| | | +++ | gut |
| **N** | "Gleitfähigkeit - Distanz" | | |
| | Im Rahmen der Gleitfähigkeit des Sondenkopfs (M) wurde die Distanz ermittelt, über die hinaus der Sondenkopf nach außerhalb des umschriebenen Flüssigkeitsfilms auf dem Flüssigkeitsfilm gleitet. | - | << 3 cm (schlecht) |
| | | + | > 3 cm |
| | | ++ | > 4 cm |
| | | +++ | > 5 cm |

### Beispiel 3

Ausgehend von der Zusammensetzung #28 (0,5% Imwitor, 0,125% Kolliphor, 0,2% Natrosol) aus Beispiel 2, Tabelle 5 wurden verschiedene Verdünnungen durch Zugabe von Wasser hergestellt (#28/Wasser: 2/1 = 66%; 1/1 = 50%; 1/2 =33%). Es wurde das Sprühverhalten im Vergleich zur unverdünnten Zusammensetzung (1/0 = 100%) beurteilt. Der pH-Wert der Zusammensetzungen betrug 5,9. Bei keiner der Zusammensetzungen wurde nach dem vorausgehenden Test A eine Schaumbildung beobachtet.

In einem weiteren Schritt wurden die Verdünnungen und die unverdünnte Zusammensetzung mit Konservierungsmittel (0,4 Gew.-% Natrium-Benzoat + 0,15 Gew.-% Kalium-Sorbat) versetzt und verschiedene Konzentrationen an Zitronensäure zugegeben und erneut der pH-Wert bestimmt. Die Zugabe des Konservierungsmittels änderte den Ausgangs-pH-Wert der Zusammensetzungen nicht. Die Ergebnisse sind in der nachfolgenden Tabelle 6 wiedergegeben.

### Beispiel 4

Die Zusammensetzung #28 (0,5% Imwitor, 0,125% Kolliphor, 0,2% Natrosol) und deren Verdünnungen aus Beispiel 3 wurden hinsichtlich Sprüheigenschaften, Filmbildung und Gleitfähigkeit entsprechend den vorausgehenden Tests J, K, M und N gemäß Beispiel 2 beurteilt. Darüber hinaus wurden Substanzverluste durch Trocknung beurteilt, indem 0,8 ml der Zusammensetzung auf einer Fläche von 20 cm² Haut verteilt und der Flächenverlust nach 1 Stunde bei Umgebungstemperatur bestimmt wurde. Die Ergebnisse sind in der nachfolgenden Tabelle 7 wiedergegeben.

### Beispiel 5

Von den Zusammensetzungen #10, 14, 17, 23, 28 wurde das spezifische Gewicht bestimmt und bei 25 °C sowie bei 37 °C die Fließfähigkeit gemäß DIN EN ISO 2431:2020-02 gemessen. Die Ergebnisse sind in der nachfolgenden Tabelle 8 wiedergegeben.

**Tabelle 5: Ausgewählte Mischungen und deren Verdünnungen**

| | Emulgiermittel | Detergens | Gelbildner | **J** | **K** | **L** | **M** | **N** | | |
|---|---|---|---|---|---|---|---|---|---|---|
| Nr. (#) | Imwitor Gew.-% | Kolliphor Gew.-% | Natrosol Gew.-% | Sprühkegel [°] | Filmbildung (Haut) | Tropfenbildung (Haut) | Gleitfähigkeit (Haut) | Gleitfähigkeit (Distanz) | Präferenz | pH-Wert |
| 23 | **1** | **0,25** | **0,2** | 20 | +++ | +++ | +++ | +++ | **X** | **5,6** |
| 24 | 1 | 0,1 | 0,4 | 0 | - | - | + | ++ | | 5,6 |
| 25 | 1 | 0,1 | 0,3 | 20 | + | +++ | ++ | + | | 5,9 |
| 26 | 0,9 | 0,45 | 0,18 | 30 | +++ | +++ | + | + | | 5,9 |
| 27 | 0,5 | 0,125 | 0,4 | 0 | +++ | +++ | + | + | | 5,9 |
| 28 | **0,5** | **0,125** | **0,2** | 30 | +++ | +++ | +++ | +++ | **X** | **5,9** |
| 29 | 0,1 | | 0,2 | 0 | - | - | ++ | + | | 5,9 |
| 30 | | | 0,3 | 0 | - | - | + | - | | 6,8 |
| Vgl. | Sonogel | | | entfällt | entfällt | entfällt | ++ | ++ | | |

**Tabelle 6: Sprühverhalten verschiedener Verdünnungen der Zusammensetzung #28**

| | Verdünnung | Emulgiermittel | Detergens | Gelbildner | **J** | **pH-Wert vor/nach Zugabe von Zitronensäure [Gew.-%]** | | | | |
|---|---|---|---|---|---|---|---|---|---|---|
| Nr. (#) | #28 / H₂O | Imwitor Gew.-% | Kolliphor Gew.-% | Natrosol Gew.-% | Sprühkegel [°] | 0% | 0,5% | 1,0% | 1,5% | 2,0% |
| 28 | 1/0 (100%) | 0,5 | 0,125 | 0,2 | 30 | 5,9 | 5,9 | 5,6-5,9 | 5,6-5,9 | 5,6 |
| 28a | 2/1 (66%) | 0,375 | 0,0932 | 0,15 | 20 | 5,9 | 5,9 | 5,9 | 5,9 | 5,6 |
| 28b | 1/1 (50% | 0,25 | 0,0625 | 0,1 | 40 | 5,9 | 5,9 | 5,9 | 5,9 | 5,6 |
| 28c | 1/2 (33%) | 0,166 | 0,0412 | 0,066 | 70 | 5,9 | 5,9 | 5,9 | 5,6 | 5,6 |

**Tabelle 7: Sprühverhalten, Filmbildung, Gleitfähigkeit und Flächenverlust verschiedener Verdünnungen der Zusammensetzung #28**

| | Verdünnung | Emulgiermittel | Detergens | Gelbildner | **J** | **K** | **M** | **N** | **Trocknung** |
|---|---|---|---|---|---|---|---|---|---|
| Nr. (#) | #28 / H₂O | Imwitor Gew.-% | Kolliphor Gew.-% | Natrosol Gew.-% | Sprühkegel [°] | Filmbildung (Haut) | Gleitfähigkeit (Haut) | Gleitfähigkeit (Distanz) | Flächenverlust |
| 28 | 1/0 (100%) | 0,5 | 0,125 | 0,2 | 30 | +++ | +++ | +++ | 10% |
| 28a | 2/1 (66%) | 0,375 | 0,0932 | 0,15 | 20 | +++ | +++ | +++ | 20% |
| 28b | 1/1 (50% | 0,25 | 0,0625 | 0,1 | 40 | +++ | +++ | +++ | 5% |
| 28c | 1/2 (33%) | 0,166 | 0,0412 | 0,66 | 70 | +++ | + | + | 0 |

**Tabelle 8: Fließfähigkeit (ISO 2431) der Zusammensetzungen #10, 14, 17, 23, 28 bei 25 °C und 37 °C**

| | Emulgiermittel | Detergens | Gelbildner | | **25°C** | **37°C** |
|---|---|---|---|---|---|---|
| Nr. (#) | Imwitor Gew.-% | Kolliphor Gew.-% | Natrosol Gew.-% | Spez. Gewicht [g/ml] | Auslaufzeit [Sek.] | Auslaufzeit [Sek.] |
| 10 | 1,0 | 2,0 | 0,4 | 1,03 | 18 | 16 |
| 14 | 1,0 | 2,0 | 0,25 | 1,02 | 13 | 13 |
| 17 | 2,0 | 2,0 | 0,4 | 1,01 | 24 | 21 |
| 23 | 1,0 | 0,25 | 0,2 | 1,00 | 14 | 13 |
| 28 | 0,5 | 0,125 | 0,2 | 1,00 | 13 | 13 |
| 28x | 0,5 | 0,125 | 0,2 | 0,97 | 10 | 10 |

Probe # 28x enthält gegenüber Probe #28 zusätzlich Konservierungsmittel (0,4 Gew.-% Natrium-Benzoat + 0,15 Gew.-% Kalium-Sorbat) und wurde mit Zitronensäure auf den pH-Wert = 5,6 eingestellt.

### Diskussion der Ergebnisse

Reines Wasser ist sehr dünnflüssig und daher auch sehr gut sprühfähig und es bildet auch keine Gasblasen aus. Im Vergleich zu den erfindungsgemäßen Kontaktmittelzusammensetzungen, welche ebenfalls sehr gut sprühfähig sind und keine oder nur geringe Gasblasenbildung zeigen, perlt Wasser aber von der Haut ab, ohne einen Flüssigkeitsfilm zu bilden. Wasser alleine ist daher als Kontaktmittel unbrauchbar.

Erst die erfindungsgemäße Kombination von Gelbildner, Detergens und Emulgator lieferte hervorragende Eigenschaften, insbesondere gute Gleiteigenschaften, gute Filmbildung, geringe bis keine Schaumbildung sowie sehr gute Schalltransmissionseigenschaften.

Insbesondere in quantitativer Hinsicht erbrachte die Kombination von Gelbildner, Detergens und Emulgator überraschend gute Ergebnisse. Erwartungsgemäß zeigten höhere Konzentrationen die guten Eigenschaften des Gemisches, orientiert an den geschilderten Kriterien. Überraschend war, dass diese Eigenschaften auch schon bei niedrigen Konzentrationen zu beobachten waren.

Ein weiterer Vorteil der Erfindung war die Sprühfähigkeit des erfindungsgemäßen Kontaktmittels im Vergleich zu herkömmlichen Kontaktmittelgelen. Das Kontaktmittel ist darüber hinaus ohne Funktionsverlust auf Körpertemperatur erwärmbar. Hierfür bieten sich Wärmevorrichtungen an, wie sie z.B. zum Warmhalten von Säuglingsmilchflaschen im Gebrauch sind. Auch andere Vorrichtungen, wie z. B. Wärmekissen sind denkbar. Im medizinischen Bereich ist das Mittel sowohl im diagnostischen als auch therapeutischen Ultraschall verwendbar und unabhängig von den verschiedenen Schallkopftypen und Ultraschalltechniken. Das erfindungsgemäße Kontaktmittel ist neben der Anwendung in der medizinischen Ultraschallanwendung auch in der der Elektrokardiografie (EKG), bei der Defibrillation sowie außerhalb des medizinischen Bereiches (z.B. technischer Ultraschall) mit Vorteil einsetzbar.

## Patentansprüche

1. Kontaktmittel, welches enthält:
| | |
|---|---|
| 0,05 bis 2,5 Gew.-% | Gelbildner |
| 0,05 % bis 3,5 Gew.-% | Detergens |
| 0,1 % bis 4,0 Gew.-% | Emulgiermittel |
optional Zusatzstoffe, vorzugsweise ausgewählt unter Konservierungsmittel, Säuerungsmittel, Hautpflegezusatz und Gemischen davon,
Rest Wasser,
wobei
der Gelbildner aus der Gruppe ausgewählt ist, bestehend aus Hydroxyethylcellulose, Carboxycellulose, Xanthan, Guarkernmehl, Collagen, Gelatine, Pektinen, Chitin, Stärke, Alginaten, einschließlich Natriumalginat, Kaliumalginat und Calciumalginat, und Gemischen davon,
das Detergens ein nicht-ionisches Tensid oder ein Gemisch von nicht-ionischen Tensiden ist,
das Emulgiermittel aus der Gruppe ausgewählt ist, bestehend aus Glycerin-Mono-Fettsäureestern und Glycerin-Di-Fettsäureestern und Gemischen davon, wobei der Fettsäurerest vorzugsweise abgeleitet ist von gesättigter C7-C20-Fettsäure, einfach ungesättigter C16-C20-Fettsäure und/oder zweifach ungesättigter C18-C20-Fettsäure, vorzugsweise von Stearinsäure, Ölsäure, Caprylsäure, Caprinsäure und/oder Linolensäure.
wobei das Kontaktmittel einen pH-Wert im Bereich von 5 bis 7 aufweist.

2. Kontaktmittel nach Anspruch 1, **dadurch gekennzeichnet, dass** es den Gelbildner in einer Menge von 0,05 bis 0,5 Gew.-% enthält.

3. Kontaktmittel nach einem der vorausgehenden Ansprüche, **dadurch gekennzeichnet, dass** > 80 Gew.-%, vorzugsweise > 90 Gew.-%, besonders bevorzugt der gesamte Gelbildner Hydroxyethylcellulose ist, vorzugsweise 2-Hydroxyethylcellulose Cellulose-2-hydroxyethyl-ether (CAS# 9004-62-0) mit einem durchschnittlichen Substitutionsgrad (DS) von 0,85 - 1,35 und/oder einem molaren Substitutionsgrad (MS) von 1,5 - 3,0.

4. Kontaktmittel nach einem der vorausgehenden Ansprüche, **dadurch gekennzeichnet, dass** > 80 Gew.-%, vorzugsweise > 90 Gew.-%, besonders bevorzugt das gesamte Detergens polyethoxyliertes Kastoröl ist.

5. Kontaktmittel nach einem der vorausgehenden Ansprüche, **dadurch gekennzeichnet, dass** das Detergens aus der Gruppe ausgewählt ist, bestehend aus polyethoxyliertem Kastoröl, Poloxameren und Gemischen davon.

6. Kontaktmittel nach einem der vorausgehenden Ansprüche, **dadurch gekennzeichnet, dass** > 80 Gew.-%, vorzugsweise > 90 Gew.-%, besonders bevorzugt das gesamte Emulgiermittel Glycerin-Mono-Fettsäureester ist.

7. Kontaktmittel nach einem der vorausgehenden Ansprüche, **dadurch gekennzeichnet, dass** es einen pH-Wert im Bereich von 5 bis 6,5, vorzugsweise im Bereich von 5 bis 6, besonders bevorzugt im Bereich von 5 bis 5,6 aufweist, wobei das Kontaktmittel optional zum Einstellen des pH-Wertes ein anorganisches oder organisches Puffersystem oder Säuerungsmittel enthält, welches vorzugsweise ausgewählt ist unter Zitronensäure, Natriumzitrat-Zitronensäure-Puffer und Phosphat-Zitrat-Puffer.

8. Kontaktmittel nach einem der vorausgehenden Ansprüche, **dadurch gekennzeichnet, dass** es Konservierungsmittel enthält, vorzugsweise Konservierungsmittel in einer Menge von 0,05 bis 1,0 Gew.-%, wobei das Konservierungsmittel besonders bevorzugt aus der Gruppe ausgewählt ist, bestehend aus Natrium-Benzoat, Kalium-Benzoat, Natrium-Sorbat, Kalium-Sorbat und Gemischen davon.

9. Kontaktmittel nach einem der vorausgehenden Ansprüche, **dadurch gekennzeichnet, dass** es weiterhin einen Hautpflegezusatz enthält, vorzugsweise Aloe Vera.

10. Kontaktmittel nach einem der vorausgehenden Ansprüche, **dadurch gekennzeichnet, dass** es eine Auslaufzeit, bestimmt nach DIN EN ISO 2431:2020-20 unter Verwendung eines Auslaufbechers mit einer 4 mm-Düse bei 25°C, im Bereich von 5 bis 200 Sekunden, vorzugsweise im Bereich von 10 bis 150 Sekunden oder im Bereich von 10 bis 100 Sekunden oder im Bereich von 10 bis 50 Sekunden aufweist.

11. Kontaktmittel nach einem der vorausgehenden Ansprüche, **dadurch gekennzeichnet, dass** es höchstens 1 Gew.-%, vorzugsweise höchstens ≤ 0,5 Gew.-% eines einwertigen Alkanols enthält, wobei das Kontaktmittel besonders bevorzug kein einwertiges Alkanol enthält.

12. Kontaktmittel nach einem der vorausgehenden Ansprüche, **dadurch gekennzeichnet, dass** es 0,1 bis 0,3 Gew.-% Gelbildner und/oder 0,075 % bis 0,5 Gew.-% Detergens und/oder 0,2 % bis 0,8 Gew.-% Emulgiermittel enthält.

13. Kit, umfassend ein Kontaktmittel nach einem der vorausgehenden Ansprüche und wenigstens einen Applikator, vorzugsweise einer Sprühflasche, und optional wenigstens einem mit Kontaktmittel befüllten Nachfüllbehälter.

14. Kit nach Anspruch 13, welches wenigstens eine Beheizvorrichtung enthält, welche für das Erwärmen des Kontaktmittels in dem Applikator und/oder in dem Nachfüllbehälter ausgelegt ist, wobei die Beheizvorrichtung vorzugsweise eine elektrische Heizeinrichtung aufweist.

15. Verwendung eines Kontaktmittels nach einem der Ansprüche 1 bis 12 als Kontaktmittel in der medizinischen und nicht-medizinischen Sonographie, in der Elektrokardiographie, in der Elektroenzephalografie und/oder in der Defibrillation durch Stromstöße, vorzugsweise in der medizinischen Sonographie.

## Claims

1. A contact medium containing:
| | |
|---|---|
| 0.05 to 2.5 wt.% | of a gel-forming agent |
| 0.05 to 3.5 wt.% | of a detergent |
| 0.1 to 4.0 wt.% | of an emulsifier |
optionally additives, preferably selected from preservatives, acidifiers, skincare additives, and mixtures thereof,
and water as the remaining element,
wherein
the gel-forming agent is selected from the group consisting of hydroxyethyl cellulose, carboxycellulose, xanthan gum, guar gum, collagen, gelatin, pectins, chitin, starch, alginates, including sodium alginate, potassium alginate, and calcium alginate, and mixtures thereof,
the detergent is a non-ionic surfactant or a mixture of non-ionic surfactants,
the emulsifier is selected from the group consisting of glycerol mono fatty acid esters and glycerol di-fatty acid esters and mixtures thereof, wherein the remaining fatty acid is preferably derived from C7-C20-saturated fatty acid, C16-C20-monounsaturated fatty acid, and/or C18-C20-diunsaturated fatty acid, preferably from stearic acid, oleic acid, caprylic acid, capric acid, and/or linolenic acid,
wherein the contact medium has a pH value in the range of 5 to 7.

2. The contact medium according to claim 1, **characterised in that** it contains the gel-forming agent in a quantity from 0.05 to 0.5 wt.%.

3. The contact medium according to any of the preceding claims, **characterised in that** > 80 wt.%, preferably > 90 wt.%, and particularly preferably the entire gel-forming agent is hydroxyethyl cellulose, preferably 2-Hydroxyethyl cellulose, Cellulose 2-hydroxyethyl ether (CAS# 9004-62-0) having an average degree of substitution (DS) of 0.85 - 1.35 and/or a molar degree of substitution of 1.5 - 3.0.

4. The contact medium according to any of the preceding claims, **characterised in that** > 80 wt.%, preferably > 90 wt.%, and particularly preferably the entire detergent is polyethoxylated castor oil.

5. The contact medium according to any of the preceding claims, **characterised in that** the detergent is selected from the group consisting of polyethoxylated castor oil, polox-amers, and mixtures thereof.

6. The contact medium according to any of the preceding claims, **characterised in that** > 80 wt.%, preferably > 90 wt.%, and particularly preferably the entire emulsifier is glycerol mono-fatty acid ester.

7. The contact medium according to any of the preceding claims, **characterised in that** it has a pH value in the range of 5 to 6.5, preferably in the range of 5 to 6, and particularly preferably in the range of 5 to 5.6, wherein the contact medium optionally contains an inorganic or organic buffer system or acidifier for adjusting the pH level, which is preferably selected from among citric acid, sodium citrate-citric acid buffer, and phosphate-citrate buffer.

8. The contact medium according to any of the preceding claims, **characterised in that** it contains preservatives, preferably preservatives in a quantity from 0.05 to 1.0 wt.%, wherein the preservative is particularly preferably selected from the group consisting of sodium benzoate, potassium benzoate, sodium sorbate, potassium sorbate, and mixtures thereof.

9. The contact medium according to any of the preceding claims, **characterised in that** it further contains a skincare additive, preferably aloe vera.

10. The contact medium according to any of the preceding claims, **characterised in that** it comprises an efflux time determined in accordance with DIN EN ISO 2431:2020-20 using an efflux beaker with a 4 mm nozzle at 25°C, in the range of 5 to 200 seconds, preferably in the range of 10 to 150 seconds or in the range of 10 to 100 seconds or in the range of 10 to 50 seconds.

11. The contact medium according to any of the preceding claims, **characterised in that** it contains at most 1 wt.%, preferably at most ≤ 0.5 wt.%, of a monovalent alkanol, wherein the contact medium particularly preferably does not contain any monovalent alkanol.

12. The contact medium according to any of the preceding claims, **characterised in that** it contains 0.1 to 0.3 wt.% of a gel-forming agent and/or 0.075 to 0.5 wt.% of a detergent and/or 0.2 to 0.8 wt.% of an emulsifier.

13. A kit comprising a contact medium according to any of the preceding claims and at least one applicator, preferably a spray bottle, and optionally at least one refilling container filled with contact medium.

14. The kit according to claim 13, containing at least one heating apparatus configured so as to heat the contact medium in the applicator and/or in the refilling container, wherein the heating apparatus preferably comprises an electric heating device.

15. A use of a contact medium according to any of claims 1 to 12 as the contact medium in medical and non-medical sonography, in electrocardiography, in electroencephalog-raphy, and/or in defibrillation by means of current impulses, preferably in medical sonography.

## Revendications

1. Agent de contact, contenant :
| | |
|---|---|
| 0,05 à 2,5 % en poids | d'agent gélifiant |
| 0,05 % à 3,5 % en poids | de détergent |
| 0,1 % à 4,0 % en poids | d'émulsifiant |
en option des additifs, de préférence choisis parmi des conservateurs, des acidifiants, des additifs de soin de la peau et leurs mélanges,
le reste étant constitué d'eau,
dans lequel
l'agent gélifiant est choisi dans le groupe constitué de l'hydroxyéthylcellulose, de la carboxycellulose, de la gomme de xanthane, de la gomme de guar, du collagène, de la gélatine, des pectines, de la chitine, de l'amidon, des alginates, y compris l'alginate de sodium, l'alginate de potassium et l'alginate de calcium, et de leurs mélanges,
le détergent est un tensioactif non ionique ou un mélange de tensioactifs non ioniques,
l'émulsifiant est choisi dans le groupe constitué de monoglycérides d'acides gras et de di-glycérides d'acides gras et de leurs mélanges, le résidu d'acide gras étant de préférence dérivé d'acides gras saturés en C7-C20, d'acides gras mono-insaturés en C16-C20 et/ou d'acides gras bi-insaturés en C18-C20, de préférence de l'acide stéarique, de l'acide oléique, de l'acide caprylique, de l'acide caprique et/ou de l'acide linolénique,
dans lequel l'agent de contact présente un pH compris entre 5 et 7.

2. Agent de contact selon la revendication 1, **caractérisé en ce qu'**il contient l'agent gélifiant en une quantité comprise entre 0,05 et 0,5 % en poids.

3. Agent de contact selon l'une des revendications précédentes, **caractérisé en ce qu'**une part > 80 % en poids, de préférence une part > 90 % en poids, de manière particulièrement préférée la totalité, de l'agent gélifiant est de l'hydroxyéthylcellulose, de préférence de la 2-hydroxyéthylcellulose ou un 2-hydroxyéthyléther de cellulose (CAS# 9004-62-0) présentant un degré de substitution moyen (DS) compris entre 0,85 et 1,35 et/ou un degré de substitution molaire (MS) compris entre 1,5 et 3,0.

4. Agent de contact selon l'une des revendications précédentes, **caractérisé en ce qu'**une part > 80 % en poids, de préférence une part > 90 % en poids, de manière particulièrement préférée la totalité, du détergent est de l'huile de ricin polyéthoxylée.

5. Agent de contact selon l'une des revendications précédentes, **caractérisé en ce que** le détergent est choisi dans le groupe constitué de l'huile de ricin polyéthoxylée, de poloxamères et de leurs mélanges.

6. Agent de contact selon l'une des revendications précédentes, **caractérisé en ce qu'**une part > 80 % en poids, de préférence une part > 90 % en poids, de manière particulièrement préférée la totalité, de l'agent émulsifiant est un monoglycéride d'acides gras.

7. Agent de contact selon l'une des revendications précédentes, **caractérisé en ce qu'**il présente un pH situé dans la plage comprise entre 5 et 6,5, de préférence dans la plage comprise entre 5 et 6, de manière particulièrement préférée dans la plage comprise entre 5 et 5,6, l'agent de contact contenant de manière optionnelle, afin d'ajuster le pH, un système tampon ou un agent d'acidification minéral ou organique, qui est de préférence choisi parmi l'acide citrique, le tampon citrate de sodium-acide citrique et le tampon phosphate-citrate.

8. Agent de contact selon l'une des revendications précédentes, **caractérisé en ce qu'**il contient un conservateur, de préférence un conservateur en une quantité comprise entre 0,05 et 1,0 % en poids, le conservateur étant choisi de manière particulièrement préférée dans le groupe constitué du benzoate de sodium, du benzoate de potassium, du sorbate de sodium, du sorbate de potassium et de leurs mélanges.

9. Agent de contact selon l'une des revendications précédentes, **caractérisé en ce qu'**il contient en outre un additif de soin de la peau, de préférence de l'Aloe vera.

10. Agent de contact selon l'une des revendications précédentes, **caractérisé en ce qu'**il présente un temps d'écoulement, déterminé selon la norme DIN EN ISO 2431: 2020-20 à l'aide d'une coupe d'écoulement présentant une buse de 4 mm à 25°C, situé dans la plage comprise entre 5 et 200 secondes, de préférence dans la plage comprise entre 10 et 150 secondes ou dans la plage comprise entre 10 et 100 secondes ou dans la plage comprise entre 10 et 50 secondes.

11. Agent de contact selon l'une des revendications précédentes, **caractérisé en ce qu'**il contient au plus 1 % en poids, de préférence au plus une part ≤ 0,5 % en poids d'un alcanol monovalent, l'agent de contact ne contenant de manière particulièrement préférée aucun alcanol monovalent.

12. Agent de contact selon l'une des revendications précédentes, **caractérisé en ce qu'**il contient entre 0,1 et 0,3 % en poids d'agent gélifiant et/ou entre 0,075 % et 0,5 % en poids de détergent et/ou entre 0,2 % et 0,8 % en poids d'émulsifiant.

13. Trousse comprenant un agent de contact selon l'une des revendications précédentes et au moins un applicateur, de préférence un flacon pulvérisateur, et en option au moins un récipient de recharge rempli d'agent de contact.

14. Trousse selon la revendication 13, comprenant au moins un dispositif de chauffage conçu pour chauffer l'agent de contact dans l'applicateur et/ou dans le récipient de recharge, le dispositif de chauffage comprenant de préférence un dispositif de chauffage électrique.

15. Utilisation d'un agent de contact selon l'une des revendications 1 à 12 comme agent de contact en échographie médicale et non médicale, en électrocardiographie, en électroencéphalographie et/ou en défibrillation par impulsions, de préférence en échographie médicale.
